(19) Europäisches Patentamt
European Patent Office
Office européen des brevets

(11) Veröffentlichungsnummer: **0 165 448 B1**

(12) # EUROPÄISCHE PATENTSCHRIFT

(45) Veröffentlichungstag der Patentschrift: **16.10.91**

(51) Int. Cl.5: **C07D 401/04**, C07D 403/04, A01N 43/48, A01N 43/54, A01N 43/56

(21) Anmeldenummer: **85105794.3**

(22) Anmeldetag: **11.05.85**

(54) **1-Heteroaryl-4-aryl-pyrozolin-5-one.**

(30) Priorität: **23.05.84 DE 3419127**
**18.08.84 DE 3430433**

(43) Veröffentlichungstag der Anmeldung:
**27.12.85 Patentblatt 85/52**

(45) Bekanntmachung des Hinweises auf die Patenterteilung:
**16.10.91 Patentblatt 91/42**

(84) Benannte Vertragsstaaten:
**AT BE CH DE FR GB IT LI NL**

(56) Entgegenhaltungen:
**DE-C- 1 112 984**
**FR-A- 2 529 786**

**HELVETIA CHIMICA ACTA, Band 49, 1966, Seiten 272-280, Zürich, CH; J. BÜCHI et al.: "Synthese und pharmakologische Eigenschaften einiger Pyridylpyrazolone-5-one"**

(73) Patentinhaber: **BAYER AG**

**W-5090 Leverkusen 1 Bayerwerk(DE)**

(72) Erfinder: **Sasse, Klaus, Dr.**
**Pützweg 13**
**W-5060 Bergisch-Gladbach 2(DE)**
Erfinder: **Brandes, Wilhelm, Dr.**
**Eichendorffstrasse 3**
**W-5653 Leichlingen(DE)**
Erfinder: **Hänssler, Gerd, Dr.**
**Heymannstrasse 40**
**W-5090 Leverkusen 1(DE)**
Erfinder: **Reinecke, Paul, Dr.**
**Steinstrasse 8**
**W-5090 Leverkusen 3(DE)**
Erfinder: **Schmitt, Hans-Georg, Dr.**
**Gustav-Freytag-Strasse 2**
**W-5090 Leverkusen 1(DE)**
Erfinder: **Paulus, Wilfried, Dr.**
**Deswatinesstrasse 90**
**W-4150 Krefeld 1(DE)**

**EP 0 165 448 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 1-Heteroaryl-4-aryl-pyrazolin-5-one, ein verfahren zu ihrer Herstellung und ihre Verwendung als Mikrobizide.

Es ist bereits bekannt, daß bestimmte heterocyclische Verbindungen, wie z.B. N-Trichlormethylthio-Phthalimid und -tetrahydrophthalimid, gute fungizide Wirkungen aufweisen (vgl. US-Patente 2 553 770, 2 553 771 und 2 553 776). Außerdem sind auch organische Schwefelverbindungen, wie beispielsweise das Zinkethylen-1,2-bis-(dithiocarbamat), fungizid gut wirksame Verbindungen (vgl. z.B. R. Wegler "Chemie der Pflanzenschutz- und Schädlingsbekämpfungsmittel" Springer Verlag, Berlin, Heidelberg, New York 1970, Band 2, S. 65 ff).

Die Wirkung dieser Verbindungen kann unter bestimmten Bedingungen, z.B. bei niedrigen Aufwandmengen und -konzentrationen, in einigen Anwendungsbereichen nicht immer völlig zufriedenstellend sein, z. B. auf dem Pflanzenschutzgebiet. Die Verwendung der bekannten Verbindungen zum Schutz technischer Materialien ist jedoch aufgrund mangelhafter Stabilität in wäßrigen Medien nicht zufriedenstellend.

Weiterhin sind 1,4-Diaryl-pyrazolin-5-one bekannt, die als Herbizide eingesetzt werden. Über eine mikrobizide Wirksamkeit ist nichts bekannt (vgl. DE-OS 2 651 008).

Es wurden neue 1-Heteroaryl-4-aryl-pyrazolin-5-one der Formel (I)

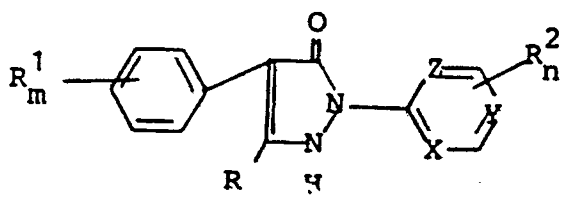

(I)

gefunden, in welcher

| | |
|---|---|
| R | für Wasserstoff, Methyl oder Ethyl steht, |
| $R^1$ | für Fluor, Chlor, Brom, Methyl, Ethyl, n-Propyl, iso-Propyl, Trifluormethyl, Dichlorfluormethyl, Methoxy, Ethoxy, n-Propoxy, iso-Propoxy, Trifluormethoxy, Dichlorfluormethoxy, Methylthiomethoxy, Ethylthiomethoxy, 1-Methylthio-ethoxy, 2-Ethylthio-ethoxy, Phenoxymethoxy, Phenoxy, Methylthio, Ethylthio, Trifluormethylthio, Dichlorfluormethylthio, Methoxymethylthio, Ethoxymethylthio, Methylthiomethylthio, Methylsulfinyl, Ethylsulfinyl, Methylsulfonyl, Nitro, Cyano, Amino, Methylamino, Ethylamino, Dimethylamino, Diethylamino, Acetylamino, für einen ankondensierten Benzolring oder einen gegebenenfalls durch Fluor einfach oder mehrfach substituierten ankondensierten 5-oder 6-gliedrigen Heterocyclus mit 1 oder 2 Sauerstoffatomen steht, |
| $R^2$ | für Fluor, Chlor, Brom, Methyl, Ethyl, n-Propyl, iso-Propyl, tert.-Butyl, Trifluormethyl, Dichlorfluormethyl, Methoxymethyl, Ethoxymethyl, Methylthiomethyl, Methoxy, Ethoxy, n-Propoxy, iso-Propoxy, Methylthio, Ethylthio, Nitro, Cyano, Carbonsäureamid oder einen ankondensierten Benzolring steht, |
| m | für eine ganze Zahl 0 bis 3 steht, |
| n | für eine ganze Zahl von 0 bis 3 steht, wobei die Substituenten gleich oder verschieden sein können, wenn m und/oder n für eine Zahl größer 1 stehen und |
| X, Y und Z | gleich oder verschieden sind und für ein Stickstoffatom, den Rest =CH- oder |

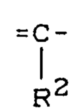

stehen, wobei $R^2$ die oben angegebene Bedeutung hat, mit der Maßgabe, daß wenigstens einer der Reste X, Y, Z ein Stickstoffatom ist.

Die Verbindungen der Formel (I) können im tautomeren Gleichgewicht mit den Verbindungen der Formel (IA) vorliegen:

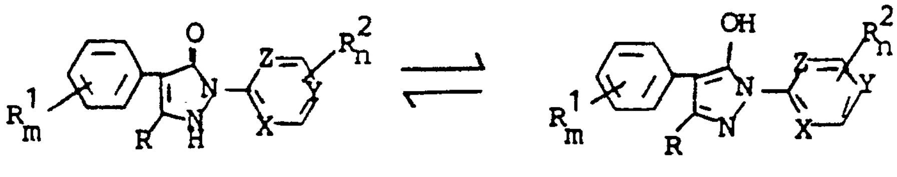

I IA

Im nachfolgenden wird der Einfachheit halber stets von Verbindungen der Formel (I) gesprochen, obwohl sowohl die reinen Verbindungen oder auch ihre Gemische mit unterschiedlichen Anteilen der Verbindungen der Formel (I) und (IA) gemeint sind.

Weiterhin wurde gefunden, daß man die neuen 1-Heteroaryl-4-aryl-pyrazolin-5-one der Formel (I)

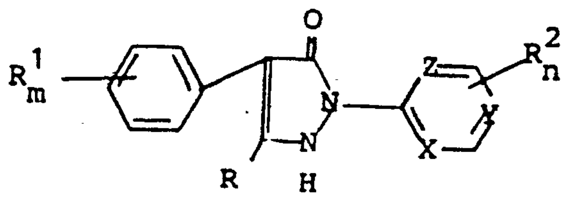

(I)

in welcher

| | |
|---|---|
| R | für Wasserstoff, Methyl oder Ethyl steht, |
| $R^1$ | für Fluor, Chlor, Brom, Methyl, Ethyl, n-Propyl, iso-Propyl, Trifluormethyl, Dichlorfluormethyl, Methoxy, Ethoxy, n-Propoxy, iso-Propoxy, Trifluormethoxy, Dichlorfluormethoxy, Methylthiomethoxy, Ethylthiomethoxy, 1-Methylthio-ethoxy, 2-Ethylthio-ethoxy, Phenoxymethoxy, Phenoxy, Methylthio, Ethylthio, Trifluormethylthio, Dichlorfluormethylthio, Methoxymethylthio, Ethoxymethylthio, Methylthiomethylthio, Methylsulfinyl, Ethylsulfinyl, Methylsulfonyl, Nitro, Cyano, Amino, Methylamino, Ethylamino, Dimethylamino, Diethylamino, Acetylamino, für einen ankondensierten Benzolring oder einen gegebenenfalls durch Fluor einfach oder mehrfach substituierten ankondensierten 5-oder 6-gliedrigen Heterocyclus mit 1 oder 2 Sauerstoffatomen steht, |
| $R^2$ | für Fluor, Chlor, Brom, Methyl, Ethyl, n-Propyl, iso-Propyl, tert.-Butyl, Trifluormethyl, Dichlorfluormethyl, Methoxymethyl, Ethoxymethyl, Methylthiomethyl, Methoxy, Ethoxy, n-Propoxy, iso-Propoxy, Methylthio, Ethylthio, Nitro, Cyano, Carbonsäureamid oder einen ankondensierten Benzolring steht, |
| m | für eine ganze Zahl 0 bis 3 steht, |
| n | für eine ganze Zahl von 0 bis 3 steht, wobei die Substituenten gleich oder verschieden sein können, wenn m und/oder n für eine Zahl größer 1 stehen und |
| X, Y und Z | gleich oder verschieden sind und für ein Stickstoffatom, den Rest =CH- oder 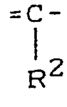 stehen, wobei $R^2$ die oben angegebene Bedeutung hat, mit der Maßgabe, daß wenigstens einer der Reste X, Y, Z ein Stickstoffatom bedeutet, |

erhält, wenn man α-Acylphenylessigsäureester oder deren Derivate der Formel (II)

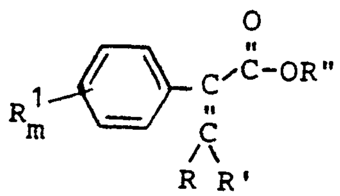

(II)

in welcher

| | |
|---|---|
| R, $R^1$ und m | die unter Formel (I) angegebenen Bedeutungen haben, |
| R' | für Hydroxy, Alkoxy, Halogen, Dialkylamino oder für die $-O-SO_2R'''$ Gruppe steht, |
| R'' | für Alkyl oder gegebenenfalls substituiertes Aryl stehen und |
| R''' | für gegebenenfalls substituiertes Alkyl, gegebenenfalls substituiertes Aryl oder Dialkylamino steht, |

mit Hydrazinoheterocyclen der Formel (III)

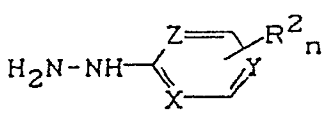

(III) ,

in welcher

| | |
|---|---|
| X, Y, Z, $R^2$ und n | die unter Formel (I) angegebenen Bedeutungen haben, gegebenenfalls in Gegenwart eines Lösungsmittels und gegebenenfalls in Gegenwart einer Base oder Säure umsetzt. |

Schließlich wurde gefunden, daß die 1-Heteroaryl-4-aryl-pyrazolin-5-oneder Formel (I) als Mikrobizide eingesetzt werden können.

Dabei zeigen die erfindungsgemäßen Verbindungen der Formel (I) überraschenderweise eine höhere und breitere mikrobizide Wirksamkeit als die aus dem Stand der Technik vorbekannten, mikrobizid wirksamen Verbindungen, wie z.B. das N-Trichlormethylthiophthalimid oder -tetrahydrophthalimid, das Zinkethylen-1,2-bis-(dithiocarbamat) und/oder das N,N-Dimethyl-N'-phenyl-N'-dichlorfluormethylthio-sulfamid. Die erfindungsgemäßen Verbindungen stellen somit eine Bereicherung der Technik dar.

Bevorzugt sind die Verbindungen der Formel (I), in denen

| | |
|---|---|
| R | für Wasserstoff, Methyl oder Ethyl steht, |
| $R^1$ | für Fluor, Chlor, Brom, Methyl, Ethyl, Trifluormethyl, Methoxy, Ethoxy, Trifluormethoxy, Phenoxy, Methylthio, Ethylthio, Trifluormethylthio, Nitro, Cyano, Amino, Dimethylamino, Acetylamino, einen ankondensierten Benzolring oder einen ankondensierten 5-gliedrigen, mehrfach durch Fluor substituierten Heterocyclus mit 2 Sauerstoffatomen steht, |
| $R^2$ | für Fluor, Chlor, Brom, Methyl, Ethyl, tert.-Butyl, Trifluormethyl, Methoxy, Methylthio, Nitro, Cyano, Carbonsäureamid oder einen ankondensierten Benzolring steht, |
| m | für eine ganze Zahl von 0 bis 3 steht, |
| n | für eine ganze Zahl von 0 bis 3 steht und |
| X | für ein Stickstoffatom steht und |
| Y und Z | für den Rest =CH- oder |

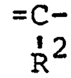

| | |
|---|---|
| | stehen, oder |
| Y | für ein Stickstoffatom steht und |
| X und Z | für den Rest =CH- oder |

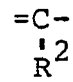

stehen oder

X und Z für je ein Stickstoffatom stehen und

Y für den Rest =CH- oder

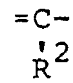

steht oder

X und Y für je ein Stickstoffatom stehen und

Z für den Rest =CH- oder

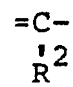

steht.

Verwendet man beispielsweise α-Hydroxymethylen-phenylessigsäureethylester und 2-Hydrazino-pyrimidin als Ausgangsstoffe, so kann der Reaktionsablauf durch das folgende Formelschema wiedergegeben werden:

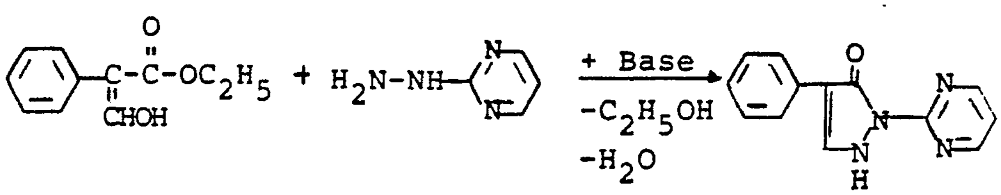


Die bei der Durchführung des erfindungsgemäßen Verfahrens als Ausgangsstoffe zu verwendenden α-Acylphenylessigsäureester oder deren Derivate sind durch die Formel (II) allgemein definiert. R, $R^1$ und m haben darin die Bedeutung, die bereits im Zusammenhang mit der Beschreibung der erfindungsgemäßen Stoffe der Formel (I) für diese Substituenten genannt wurden.

R' steht darin bevorzugt für Hydroxy, Alkoxy mit 1 bis 3 Kohlenstoffatomen, für Chlor, Brom, Dimethylamino, Methylsulfonyloxy, Trifluormethylsulfonyloxy oder für Phenylsulfonyloxy und

R" steht bevorzugt für Alkyl mit 1 bis 3 Kohlenstoffatomen oder gegebenenfalls durch Nitro substituiertes Phenyl.

Die α-Acylphenylessigsäureester oder deren Derivate der Formel (II) sind grundsätzlich bekannt. Falls die eine oder andere Verbindung noch nicht bekannt sein sollte, kann sie analog den folgenden Methoden hergestellt werden.

Für den Fall, daß R ein Wasserstoffatom darstellt, gewinnt man diese aus entsprechend substituierten Phenylessigsäurealkylestern durch Umsetzung mit einem Ameisensäurederivat. So werden Verbindungen II mit R' für Hydroxy hergestellt durch Einwirkung von Ameisensäuremethyl- oder -ethylester in Gegenwart starker Basen wie Alkalialkoholate, Natriumamid oder dgl. (vgl. z.B. B. 20, 2931 (1887); B. 28, 771 (1895); Ann. 291, 164 (1896)), wobei zunächst die Alkalisalze (II; R' = O-Metall) entstehen, die auch direkt in die Folgereaktion eingesetzt werden können oder aber durch Behandeln mit wäßrigen Säuren in die freien Hydroxymethylenverbindungen übergehen, die im tautomeren Gleichgewicht mit den entsprechenden α-Formyl-phenylessigestern stehen.

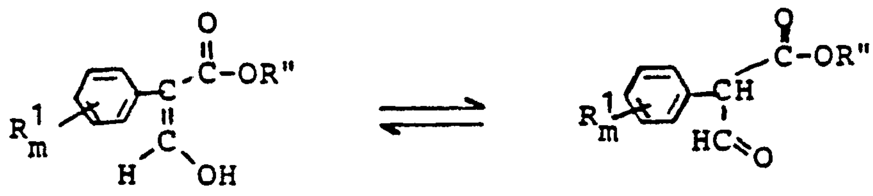

IIa

Verbindungen mit R' = OAlk werden literaturgemäß hergestellt durch Einwirkung von Alkylierungsmitteln auf Verbindungen II mit R' = OH unter basischen Bedingungen (vgl. z.B. Ann. 424, 228 (1921); J. Chem. Soc. 1953, 3548; J. org. Chem. 45, 2576 (1980)) oder durch Veretherung der gleichen Verbindungen mit Alkoholen in Gegenwart von p-Toluol-sulfonsäure (vgl. z.B. J. chem. Soc. 1953, 3548).

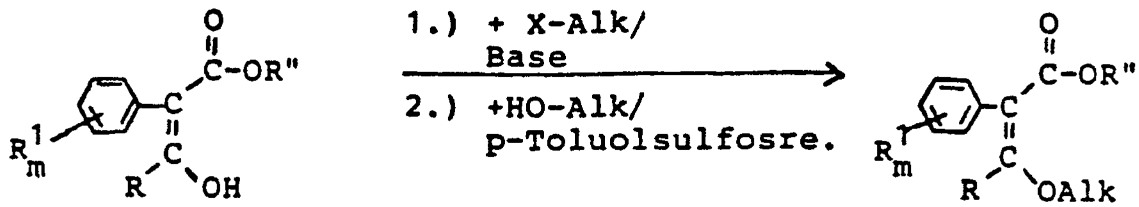


II a

II b

Verbindungen II mit R' = R'''-$SO_2$-O-Gruppe lassen sich aus den entsprechenden Verbindungen II mit R' = OH durch Umsetzung mit Sulfonsäurechloriden, wie Methansulfochlorid, Trifluormethansulfochlorid oder p-Toluolsulfonsäurechlorid, in Gegenwart von Alkalien herstellen:

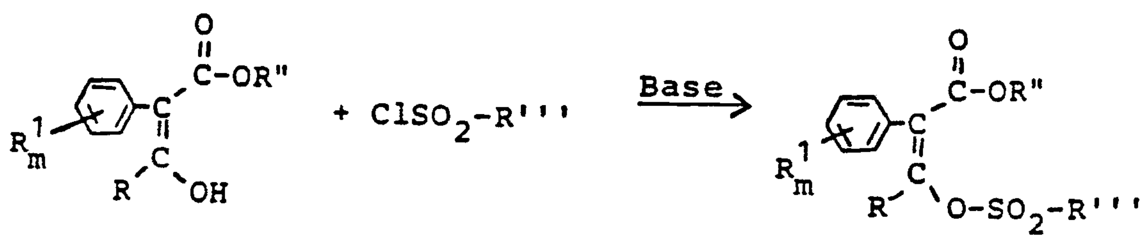


II c

Verbindungen II mit R' = Hal werden gewonnen, indem man die Hydroxymethylenverbindungen (II) mit R' = OH mit anorganischen Säurechloriden, vorzugsweise Phosphor(V)-chlorid, umsetzt (vgl. z.B. B. 51, 1366 (1918)).

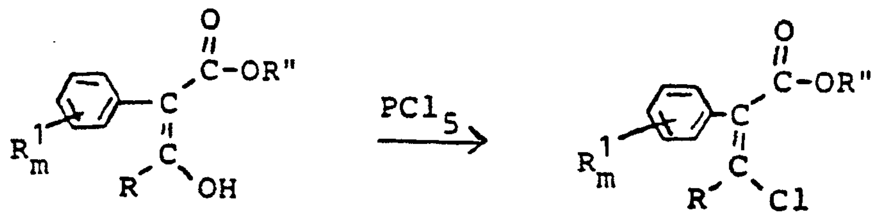


II .a

II d

Verbindungen II mit R' = Dialkylamino werden gewonnen, indem die entsprechend substituierten Phenylessigsäurealkylester mit Dialkylformamid-dialkylacetalen umgesetzt werden (vgl. Tetrahedron Lett. 16, 1361 (1979)), z.B.

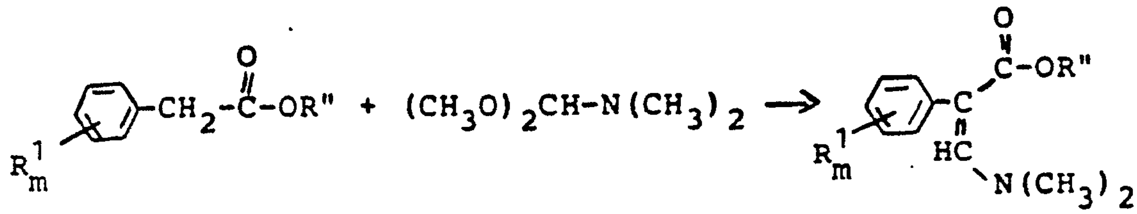

II e

Eine weitere Methode zur Herstellung dieser Verbindungen (II e) besteht in der Umsetzung der Hydroxy-methylenderivate (II a) mit sekundären Aminen (vgl. A. ch. [10] 18, 103, 114 (1932)).

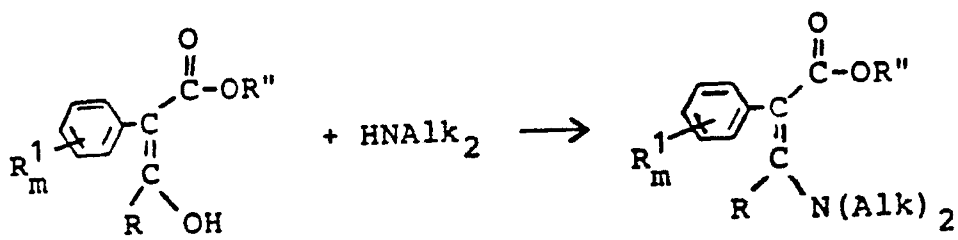

II a

II e

Zur Herstellung der Verbindungen der Formel II, in denen R einen niederen Alkylrest darstellt, lassen sich prinzipiell die gleichen Methoden anwenden wie zur Herstellung der Verbindungen mit R = Wasserstoff, jedoch verlaufen diese häufig in unzureichenden Ausbeuten. Ergiebiger verläuft deren Synthese, wenn man die Acylierung nicht mit den Phenylessigsäureestern, sondern mit Phenylacetonitrilen durchfuhrt und nachträglich die Nitrilgruppe in die Estergruppe umwandelt:

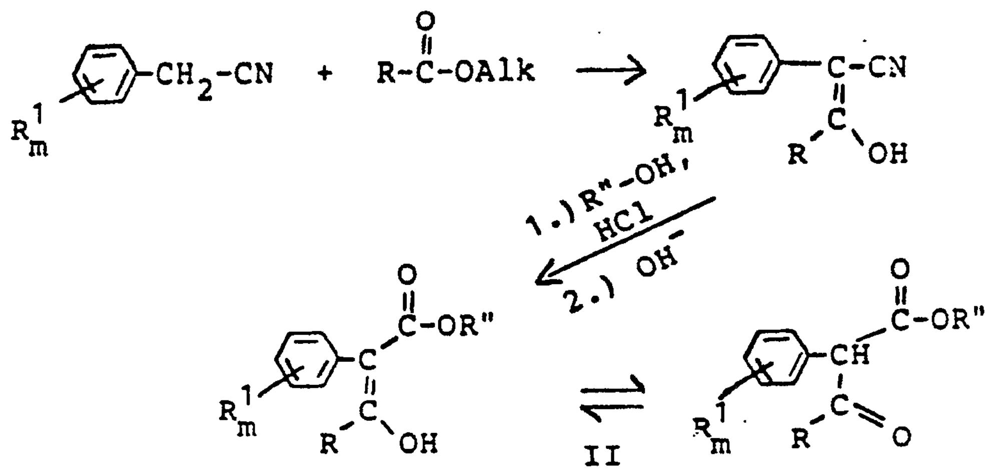


Die weiterhin zur Herstellung der erfindungsgemäßen Verbindungen der Formel I benötigten Vorprodukte der Formel III sind grundsätzlich bekannt. Es handelt sich um Hydrazinopyridine, Hydrazino-pyrimidine, Hydrazino-1,3,5-triazine und den entsprechenden Verbindungen mit ankondensierten Ringen:

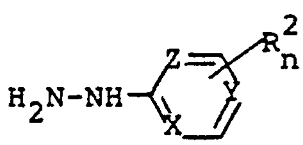

(III)

X, Y, Z, $R^2$ und n haben darin die Bedeutung, die bereits im Zusammenhang mit der Beschreibung der erfindungsgemäßen Stoffe der Formel (I) für diese Substituenten genannt wurden.

Die Herstellung der Verbindungen III ist grundsätzlich bekannt. Bisher nicht vorbeschriebene Varianten von III lassen sich auf analogen Wegen herstellen, insbesondere

a) durch Umsetzung von Halogen-N-heterocyclen, Alkoxy- oder Alkylmercapto-N-heterocyclen mit Hydrazin:

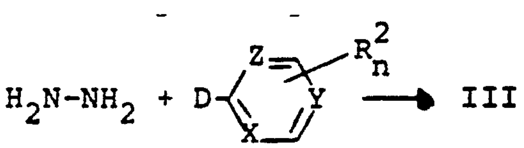

D = Hal, OAlk, SAlk

oder

b) durch Reduktion von heterocyclischen Diazoniumverbindungen:

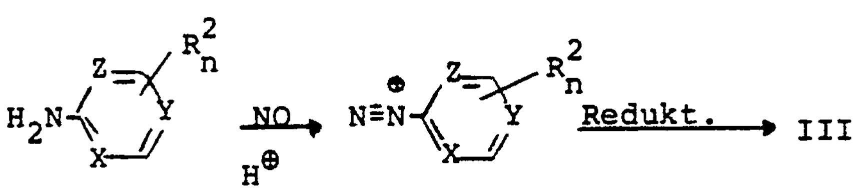


oder

c) durch Reduktion von heterocyclischen Nitraminoverbindungen:

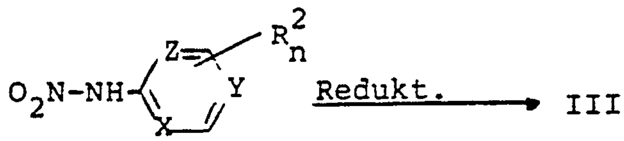


Als Beispiele für Zwischenprodukte der Formel III seien genannt:

| | Fp(°C)/Kp. | Literatur |
|---|---|---|
| 2-Hydrazino-pyridin | $Kp_{12}$: 130 | J. Chem. Soc. 107, 691 (1915) |
| 5-Chlor-2-hydrazino-pyridin | 127-128 | |
| 6-Chlor-2-hydrazino-pyridin | 116-117 | |
| 3,5-Dichlor-2-hydrazino-pyridin | 172-174 | |
| 5-Brom-2-hydrazino-pyridin | 133-134 | |
| 3-Nitro-2-hydrazino-pyridin | 200 | B. 57, 1192 (1924) |
| 5-Nitro-2-hydrazino-pyridin | 204 (Z.) | E.P. 2471488 |
| 5-Cyan-2-hydrazino-pyridin | 185-186 | |
| 2-Hydrazino-4-methyl-pyridin | 74-75 | Ann. 656, 103 (1962) |
| 2-Hydrazino-6-methyl-pyridin | HCl-Salz: 194 (Z.) | |
| 4-Chlor-2-hydrazino-6-methyl-pyridin | 162-163 | |
| 3-Nitro-2-hydrazino-6-methyl-pyridin | 138-139 | J. am. Chem. Soc. 74, 3828 (1952) |
| 5-Nitro-2-hydrazino-6-methyl-pyridin | 119-121 | |
| 2-Hydrazino-4,6-dimethyl-pyridin | | |
| 3-Cyano-2-hydrazino-6-methyl-pyridin | 243 (Z.) | |
| 2-Hydrazino-5-trifluormethyl-pyridin | 68-69 | |
| 4-Hydrazino-pyridin | $Kp_{18}$: 187 | B. 59, 317 (1926) |

| | Fp(°C)/Kp. | Literatur |
|---|---|---|
| 4-Hydrazino-2,6-dimethyl-pyridin | | B. 31, 2497 (1898) |
| 3-Chlor-4-hydrazino-pyridin | | |
| 2-Hydrazino-chinolin | 142-143 | J. Chem. Soc. 103, 1978 (1913) |
| 2-Hydrazino-4-methyl-chinolin | 145-146 | |
| 4-Hydrazino-chinolin | | |
| 1-Hydrazino-isochinolin | 172 | Ann. 656, 103 (1962) |
| 2-Hydrazino-pyrimidin | 110-111 | |
| 5-Fluor-2-hydrazino-pyrimidin | 140-141 | |
| 5-Chlor-2-hydrazino-pyrimidin | 183-184 | |
| 4-Methoxy-2-hydrazino-pyrimidin | | |
| 2-Hydrazino-4-methyl-pyrimidin | 85-86 | |
| 2-Hydrazino-4,6-dimethyl-pyrimidin | 165 | J. chem. Soc. 1952, 4691 |
| 4-Methoxy-2-hydrazino-6-methyl-pyrimidin | | |
| 4-Hydrazino-pyrimidin | | |
| 4-Hydrazino-5-methyl-pyrimidin | 205-206 | Bl. Soc. chim. Belg. 68, 30, 32 (1959) |
| 4-Hydrazino-6-methyl-pyrimidin | 140-141 | B. 34, 1241 (1901) |
| 4-Hydrazino-2,6-dimethyl-pyrimidin | 192-193 | Bl. Soc. chim. Belg. 68, 30, 32 (1959) |

| | Fp(°C)/Kp. | Literatur |
|---|---|---|
| 6-Chlor-4-hydrazino-2-methyl-pyrimidin | 152 (Z) | |
| 1-Methoxy-4-hydrazino-6-methyl-pyrimidin | | |
| 5-Methoxy-4-hydrazino-2-methyl-pyrimidin | | |
| 5-Methoxy-4-hydrazino-2-tert.butyl-pyrimidin | 213-214 | |
| 2-Methylmercapto-4-hydrazino-pyrimidin | 143-144 | |
| 2-Methylmercapto-4-hydrazino-6-methyl-pyrimidin | 142-143 | |
| 2-Hydrazino-4,6-dimethyl-1,3,5-triazin | | |
| 4-Methoxy-2-hydrazino-6-methyl-1,3,5-triazin | | |
| 4-Methylmercapto-2-hydrazino-6-methyl-1,3,5-trazin | | |
| 4,6-Dimethoxy-2-hydrazino-1,3,5-triazin | 121 | |

Die Umsetzung der substituierten Atropasäureester der Formel (II) mit den Hydrazino-N-heterocyclen der Formel (III) zu den erfindungsgemäßen 1-Heteroaryl-4-aryl-pyrazolin-5-onen der Formel (I) vollzieht sich in zwei Stufen, indem zunächst die endständige Amino-Gruppe des heterocyclischen Hydrazins mit der Enol-, Enamin- bzw. Halogenvinylgruppe in II zu den En-hydrazinen bzw. Hydrazonen Ia in Reaktion tritt und nachfolgend unter Abspaltung des in II gebundenen Alkohols oder Phenols der Ringschluß zu I erfolgt:

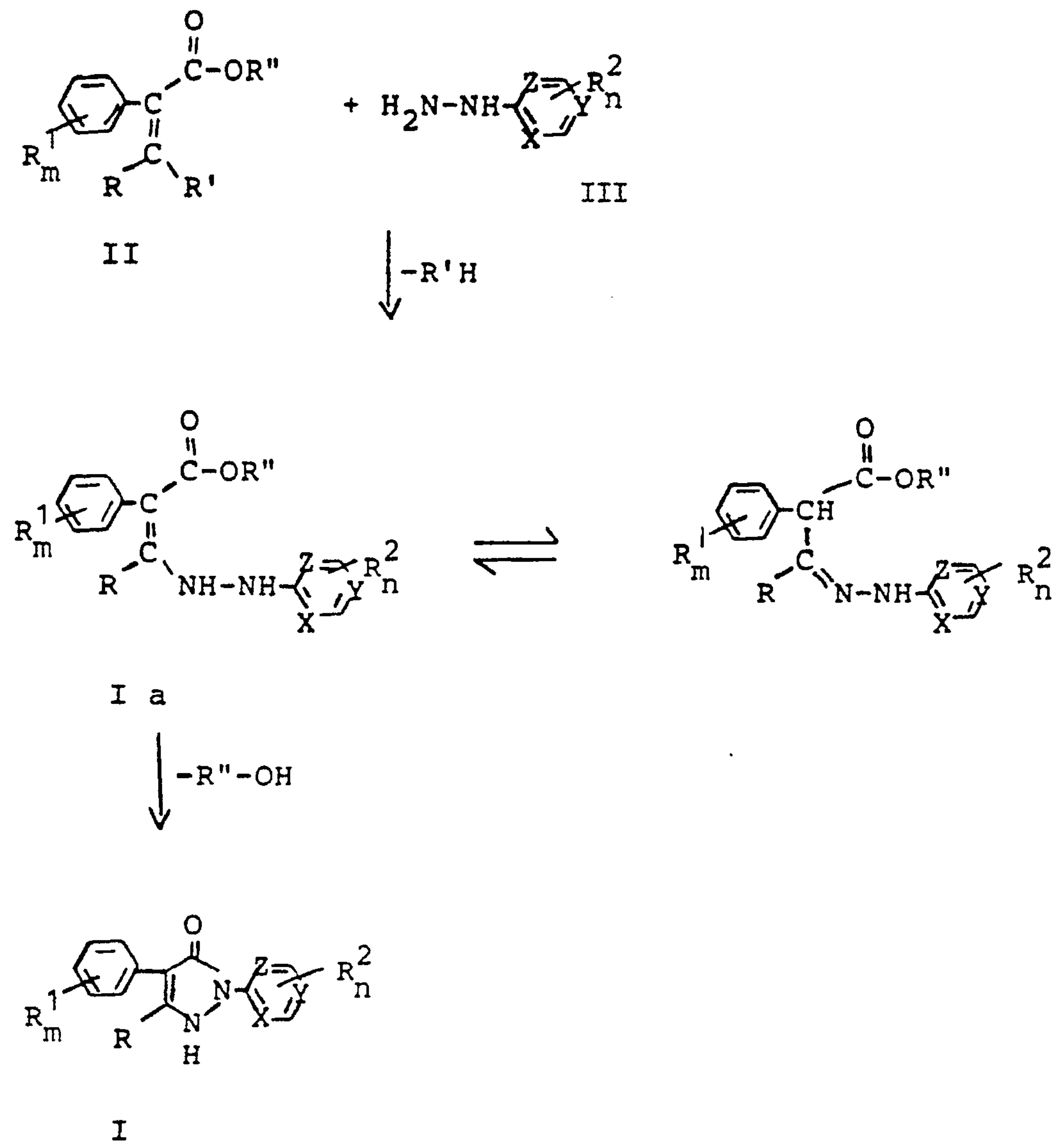


II

III

I a

I

Die Zwischenstufen Ia können isoliert und in reiner Form in die Folgereaktion eingesetzt werden. Zweckmäßigerweise führt man aber beide Stufen gleichzeitig oder nacheinander im gleichen Ansatz durch.

Die Umsetzung der Vorprodukte II und III miteinander zu den erfindungsgemäßen Verbindungen der Formel I kann ohne Lösungsmittel durch Erhitzen der Komponenten auf Temperaturen zwischen 50 und 150 °C erfolgen.

Zweckmäßigerweise nimmt man die Reaktionen in Verdünnungsmitteln vor, wobei alle gegenüber den Reaktionspartnern inerten Lösungsmittel verwendet werden können. Hierzu gehören Kohlenwasserstoffe, wie Benzin oder Toluol, Halogen-Kohlenwasserstoffe, wie Di-, Tri- und Tetrachlormethan, Alkohole, wie Methanol, Ethanol und Isopropanol, Ether, wie Diethylether, Tetrahydrofuran oder Dioxan, ferner Dimethylsulfoxid, Tetrahydrothiophendioxid, Dimethylformamid. Die Umsetzungen können auch in Wasser oder in Gemischen der genannten Lösungsmittel mit Wasser durchgeführt werden.

Die Reaktionstemperaturen können in einem größeren Bereich variiert werden. Im allgemeinen arbeitet man zwischen etwa 0 und 100 °C, vorzugsweise zwischen Raumtemperatur und dem Siedepunkt des verwendeten Lösungsmittels.

In vielen Fällen entstehen die Verbindungen I aus II und III unter den genannten Bedingungen ohne weitere Zusätze. In Abhängigkeit von der Abgangsgruppe R' in II empfiehlt sich aber häufig das Arbeiten unter Zusatz von Basen oder Säuren. Der Zusatz von Basen ist zweckmäßig, wenn R' eine säureliefernde Abgangsgruppe darstellt, z.B. ein Halogenatom wie in IId oder eine R'''-$SO_2$-O-Gruppe wie in IIc. In diesen Fällen wendet man vorzugsweise die äquimolare Menge einer Base an. Als solche sind geeignet Alkali- und Erdalkalihydroxide und -carbonate, Alkoholate oder tertiäre Amine, wie z.B. Triethylamin und Pyridin.

Der Zusatz von Säuren ist zweckmäßig, wenn die Abgangsgruppe R' eine basenliefernde Abgangsgruppe darstellt, z.B. eine Dialkylaminogruppe wie in IIe. In diesen Fällen wendet man vorzugsweise die äquimolare Menge einer Säure an, z.B. Mineralsäuren, wie Chlorwasserstoff oder Schwefelsäure, oder eine organische Säure, wie Essigsäure. Man kann hierbei auch so vorgehen, daß man die Enamine IIb, z.B. durch Behandeln mit der äquimolaren Menge trockenem Chlorwasserstoff in inerten Lösungsmitteln in ein Salz überführt und diese zum Einsatz bringt.

Das Arbeiten im sauren Medium kann aber auch beim Einsatz von Verbindungen der Formel II mit R' = OH (IIa) oder R' = OAlk zweckmäßig sein, wobei in der Regel untermolare Mengen von 0,1 bis 0,2 Mol genügen. Anstelle des nachträglichen Zusatzes von Säure kann man jedoch auch so vorgehen, daß man die Hydrazinoheterocyclen der Formel III nicht als freie Basen sondern in Form ihrer Salze, z.B. der Hydrochloride, in die Reaktion einbringt.

Diese Vorgehensweise ist insbesondere dann empfehlenswert, wenn die Hydrazine, wie das 2-Hydrazino-pyridin und das 4-Hydrazino-pyridin, als freie Basen zur Zersetzung neigen.

Beim Arbeiten im sauren Medium bleibt die Reaktion in der Regel auf der nicht cyclisierten Stufe Ia stehen. Für den Ringschluß zu den erfindungsgemäßen Verbindungen I ist ein neutrales, günstigerweise basisches Medium notwendig. Falls die erste Stufe der Reaktion im sauren Medium durchgeführt wird, ist daher der Zusatz einer Base erforderlich. Als solche kommen vorzugsweise in Betracht: Alkali- und Erdalkalicarbonate und -hydroxide, sowie Alkali- und Erdalkali-alkoholate, sowie Alkaliamide (vorzugsweise beim Arbeiten im wasserfreien Medium).

Die Base wird mindestens in der zur Säuremenge äquivalenten Menge angewandt. Ein Überschuß bis zu einem weiteren Mol kann zweckmäßig sein, jedoch sollte das Medium einem pH-Wert von mindestens 9 entsprechen. Die Cyclisierungsstufe (Ia → I) wird bei einer Temperatur zwischen 0 °C und dem Siedepunkt des angewandten Lösungsmittels, vorzugsweise zwischen 5 und 100 °C durchgeführt.

Im alkalischen Medium treten oxidable Zwischenstufen auf, die sich durch eine Gelb- bis Violettfärbung zu erkennen geben. Daher empfiehlt es sich, die Durchführung der Cyclisierungsreaktion im basischen Medium in einer Inertgasatmosphäre, z.B. unter Stickstoff, vorzunehmen.

Die erfindungsgemäßen Verbindungen der Formel I bilden mit Basen salzartige Verbindungen. Daher wird die Aufarbeitung der Reaktionsansätze in der Regel so vorgenommen, daß man vor Isolierung der Reaktionsprodukte die der eingesetzten Base mindestens äquivalente Menge einer Säure, z.B. Salzsäure, Schwefelsäure oder Essigsäure, zusetzt. Man kann auch so vorgehen, daß man die in der Regel aus dem Reaktionsmedium ausfallenden Alkali- bzw. Erdalkalisalze durch Filtration oder Absaugen aus dem Reaktionsgemisch entfernt und nachträglich mit der mindestens äquivalenten Menge einer wäßrigen Säure behandelt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind u.a. für den Gebrauch als Pflanzenschutzmittel geeignet, vor allem als Fungizide, ebenso als Wirkstoffe zur Bekämpfung von Mikroorganismen in technischen Materialien.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Botrytis-Arten, wie beispielsweise Botrytis cinerea;
Plasmopara-Arten, wie beispielsweise Plasmopara viticola,
Uromyces-Arten, wie beispielsweise Uromyces appendiculatus;
Sphaerotheca-Arten, wie beispielsweise Sphaerotheca fuliginea;
Venturia-Arten, wie beispielsweise Venturia inaequalis;
Podosphaera-Arten, wie beispielsweise Podosphaera leucotricha;
Phytophthora-Arten, wie beispielsweise Phytophthora infestans;
Erysiphe-Arten, wie beispielsweise Erysiphe graminis;
Puccinia-Arten, wie beispielsweise Puccinia recondita;
Fusarium-Arten, wie beispielsweise Fusarium culmorum;
Ustilago-Arten, wie beispielsweise Ustilago nuda oder avenae,
Septoria-Arten, wie beispielsweise Septoria nodorum;
Tilletia-Arten, wie beispielsweise Tilletia caries;
Pyricularia-Arten, wie beispielsweise Pyricularia oryzae;
Pellicularia-Arten, wie beispielsweise Pellicularia sasakii;
Pyrenophora-Arten, wie beispielsweise Pyrenophora teres (Konidienform: Drechslera, Syn: Helminthosporium);
Leptosphaeria-Arten, wie beispielsweise Leptosphaeria nodorum;

Cochliobolus-Arten, wie beispielsweise Cochliobolus sativus
(Kondienform: Drechslera, Syn: Helminthosporium)
und
Cerospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Obst- und Gemüsekrankheiten, wie beispielsweise gegen den Erreger der Braunfäule (Phytophthora infestans) an Tomaten und den Erreger der Grauschimmelfäule (Botrytis cinerea) an Bohnen, eingesetzt werden; weiterhin zur Bekämpfung von Reiskrankheiten, wie beispielsweise gegen den Erreger der Blattfleckenkrankheit (Pyricularia oryzae); außerdem zur Bekämpfung von Getreidekrankheiten, zum Beispiel verursacht durch Leptosphaeria nodorum, Cochliobolus sativus und Drechslera graminea. Außerdem sei die fungizide Wirkung gegen echte Mehltaupilze an Gurken und Getreide, gegen den Apfelschorf, gegen Rost und Pyrenophora teres an Getreide erwähnt. Außerdem sei die bakterizide Wirkung der Verbindungen erwähnt.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben,

Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die erfindungsgemäßen 1-Heteroaryl-4-aryl-pyrazolin-5-one können, wie schon erwähnt, auch als Wirkstoffe zur Bekämpfung von Mikroorganismen in technischen Materialien verwendet werden.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Papiere Leder, Holz, Anstrichmittel, Kühlschmiermittel, Textilien und Kunststoffe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeroginosa,
Staphylococcus, wie Staphylococcus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel zum Schutz technischer Materialien enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen

vorliegen. Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthaldinitril, Thiazolylbenzimidazol, Mercaptobenzthiazol, Organo-Zinnverbindungen, Methylenbisthiocyanat, Phenolderivate, wie 2-Phenylphenol, (2,2'-Dihydroxy-5,5'-dichlor)-diphenylmethan und 3-Methyl-4-chlor-phenol.

Herstellungsbeispiele:

Beispiel 1

Variante 1a:

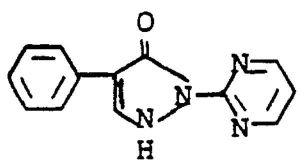

19,2 g (0,1 Mol) α-Hydroxymethylen-phenylessigsäureethylester und 11 g (0,1 Mol) 2-Hydrazino-pyrimidin werden in 150 ml Ethanol 3 Stunden unter Rückfluß gekocht. Das Gemisch wird auf Raumtemperatur abgekühlt und anschließend tropfenweise unter Rühren mit 9 g (0,1 Mol) konz. Natronlauge versetzt. Man rührt 2 Stunden bei Raumtemperatur nach und kocht anschließend 2 Stunden unter Rückfluß. Das Gemisch wird mit konz. Salzsäure neutralisiert und mit 1 l Wasser verdünnt. Die abgeschiedenen Kristalle werden abgesaugt und an der Luft getrocknet. Man erhält 15 g (63 % der Theorie) 1-Pyrimidyl-(2)-4-phenyl-pyrazolin-5-on mit einem Schmelzpunkt 159 bis 160 ° C (aus Ethanol).

Variante 1b:

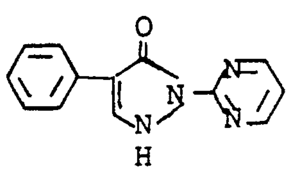

20,6 g (0,1 Mol) α-Methoxymethylen-phenylessigsäureethylester ($Kp_{0,2}$: 103 bis 106 ° C) werden mit 11 g (0,1 Mol) 2-Hydrazino-pyrimidin in 100 ml Dioxan 24 Stunden unter Rückfluß erhitzt. Das Lösungsmittel wird im Vakuum abdestilliert und der Rückstand in 50 ml Toluol verrührt. Dabei scheiden sich 3,2 g (13,5 % der Theorie) 1-Pyrimidyl-(2)-4-phenyl-pyrazolin-5-on ab, die abgesaugt und getrocknet werden. Sie haben einen Schmelzpunkt von 159 bis 160 ° C (aus Ethanol).

Variante 1c:

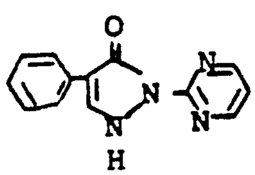

19,2 g (0,1 Mol) α-Hydroxymethylen-phenylessigsäureethylester werden in 100 ml Acetonitril gelöst und zunächst mit 11,2 g (0,1 Mol) Kalium-tert.-butylat, dann bei 20 bis 25 ° C portionsweise mit 19 g (0,1 Mol) p-Toluolsulfochlorid versetzt. Man rührt 4 Stunden bei Raumtemperatur und läßt über Nacht stehen. Das

abgeschiedene Salz wird abgesaugt und das Filtrat im Vakuum eingedampft. Der Rückstand wird in Toluol gelöst, die Lösung mit Wasser gewaschen, über Natriumsulfat getrocknet und im Vakuum eingedampft. Man erhält 23 g (73 % der Theorie) α-(4-Methyl-phenyl-sulfonyloxymethylen)-phenylessigsäureethylester. Das Öl wird in 150 ml Ethanol gelöst und dann mit 7,7 g (0,07 Mol) 2-Hydrazino-pyrimidin versetzt. Das Gemisch wird 10 Stunden bei Raumtemperatur gerührt und dann im Verlaufe von 4 Stunden tropfenweise mit 13,5 g konz. Natronlauge versetzt. Nach weiterem 5-stündigem Rühren bei Raumtemperatur wird verd. Salzsäure bis zum Neutralpunkt zugetropft und mit 750 ml Wasser verdünnt. Das sich abscheidende ölige Produkt wird in wenig Ether aufgenommen, wobei sich 3,6 g (22 % der Theorie) 1-Pyrimidyl-(2)-4-phenyl-pyrazolin-5-on abscheiden. Schmelzpunkt 159 bis 160 °C (aus Ethanol).

Beispiel 2

Variante 2a:

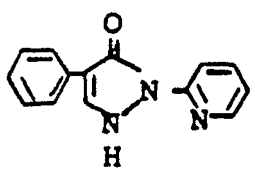

22,7 g (0,1 Mol) des Kaliumsalzes vom α-Hydroxymethylenphenylessigsäure-ethylester und 18,2 g (0,1 Mol) 2-Hydrazino-pyridin-dihydrochlorid werden in 200 ml Ethanol 30 Minuten bei Raumtemperatur verrührt und anschließend 5 Stunden unter Rückfluß gekocht. Nach dem Abkühlen auf Raumtemperatur werden unter gleichzeitigem Überleiten von Stickstoff portionsweise 19,1 g (1,7 Mol) Kalium-tert.-butylat eingetragen. Die Mischung wird 4 Stunden bei Raumtemperatur gerührt und über Nacht stehen gelassen. Der gebildete Niederschlag wird abgesaugt, in 100 ml Wasser angeschlämmt und mit Essigsäure schwach angesäuert. Die Kristalle werden abgesaugt, mit Wasser gewaschen und an der Luft getrocknet. Man erhält 17,2 g (72,5 % der Theorie) 1-Pyridyl-(2)-4-phenyl-pyrazolin-5-on mit einem Schmelzpunkt von 132 bis 133 °C (Ethanol).

Variante 2b:

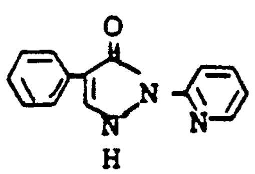

21,2 g (0,1 Mol) Phenylessigsäure-phenylester werden mit 11,9 g (0,1 Mol) Dimethylformamid-dimethylacetal 5 Stunden auf 100 °C erhitzt. Dann werden bei dieser Temperatur im Wasserstrahlvakuum alle flüchtigen Bestandteile abdestilliert. Der im wesentlichen aus α-Dimethylaminomethylen-phenylessigsäurephenylester bestehende Rückstand wird ohne weitere Reinigung in 100 ml Ethanol gelöst, die Lösung mit 18,2 g (0,1 Mol) 2-Hydrazinopyridin-dihydrochlorid versetzt und 5 Stunden unter Rückfluß gekocht. Nach dem Abkühlen scheiden sich 6,4 g (27 % der Theorie) des Reaktionsproduktes ab. Dieses wird abgesaugt und getrocknet. Die verbleibende ethanolische Lösung wird bei Raumtemperatur unter Stickstoff portionsweise mit 18,2 g (0,16 Mol) Kalium-tert.-butylat versetzt. Nach 5-stündigem Rühren bei Raumtemperatur wird der Niederschlag abgesaugt, in 100 ml Wasser angeschlämmt und mit Essigsäure angesäuert. Die Kristalle werden abgesaugt, mit Wasser gewaschen und an der Luft getrocknet. Dabei erhält man weitere 8,2 g des Reaktionsproduktes. Die Gesamtausbeute an 1-Pyridyl-(2)-4-phenyl-pyrazolin-5-on beträgt 14,6 g (61,6 % der Theorie).

Beispiel 3

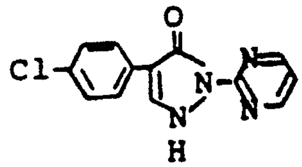

19,9 g (0,1 Mol) 4-Chlor-phenylessigsäure-ethylester werden mit 23,8 g (0,2 Mol) Dimethylformamid-dimethylacetal 5 Stunden auf 100 °C erhitzt. Anschließend werden im Wasserstrahlvakuum bei dieser Temperatur alle flüchtigen Bestandteile abdestilliert. Der im wesentlichen aus α-Dimethylaminomethylen-4-chlor-phenylessigsäureethylester bestehende ölige Rückstand wird ohne weitere Reinigung in 140 ml Ethanol gelöst und 11 g (0,1 Mol) 2-Hydrazino-pyrimidin und 10 ml konz. Salzsäure hinzugegeben. Das Gemisch wird 5 Stunden unter Rückfluß erhitzt, auf Raumtemperatur abgekühlt und nach Zugabe von 9 g konz. Natronlauge 5 Stunden bei Raumtemperatur verrührt. Nach Neutralisation mit verd. Salzsäure wird mit 1 l Wasser verdünnt. Die abgeschiedenen Kristalle werden abgesaugt, mit Wasser gewaschen und an der Luft getrocknet. Man erhält 7,2 g (26,4 % der Theorie) 1-Pyrimidyl-(2)-4-(4-chlor-phenyl)-pyrazolin-5-on mit einem Schmelzpunkt von 190 bis 191 °C (Methanol).

Die folgenden Verbindungen können nach den vorgehend beschriebenen Vorschriften hergestellt werden. Zum Umkristallisieren wurden die folgenden Lösungsmittel verwendet, auf die jeweils in der letzten Spalte der folgenden Tabellen Bezug genommen wird:

a Ethanol
b Butanol
c Toluol
d Methanol
e Essigester
f Dioxan
g Waschbenzin
h Dimethylformamid
i Glykolmonomethylether

Tabelle 1

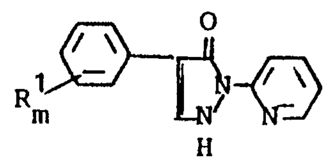

| Beisp.-Nr. | $R^1$ | m | Schmelzpunkt (°C) | Umkristallisieren aus |
|---|---|---|---|---|
| 4 | - | 0 | 131-133 | a |
| 5 | 2-F | 1 | 128-130 | g |
| 6 | 4-F | 1 | 137-139 | a |
| 7 | 2-Cl | 1 | 109-111 | a |
| 8 | 3-Cl | 1 | 146-147 | a |
| 9 | 4-Cl | 1 | 175-177 | b |
| 10 | 2-Br | 1 | 108 | b |
| 11 | 2-F, 6-Cl | 2 | 167-168 | b |
| 12 | 2,4-$Cl_2$ | 2 | 168-169 | b |
| 13 | 3,4-$Cl_2$ | 2 | 184-185 | b |
| 14 | 2-$OCH_3$ | 1 | 110-112 | a |
| 15 | 4-$OCH_3$ | 1 | 104-106 | a |
| 16 | 3-$OC_6H_5$ | 1 | 148-149 | b |
| 17 | 3-$OCF_3$ | 1 | 114-116 | a |

Tabelle 1 (Fortsetzung)

| Beisp.-Nr. | $R^1$ | m | Schmelzpunkt (°C) | Umkristallisieren aus |
|---|---|---|---|---|
| 18 | $4-OCF_3$ | 1 | 120-122 | g |
| 19 | $4-SCF_3$ | 1 | 186-188 | a |
| 20 | $-O-CF_2-O-$ (3,4-Stellg.) | 2 | 179-181 | c |
| 21 | $2-CH_3$ | 1 | 86- 88 | b |
| 22 | $3-CH_3$ | 1 | 154-156 | a |
| 23 | $4-CH_3$ | 1 | 132-133 | a |
| 24 | $3-CF_3$ | 1 | 130-131 | a |
| 25 | $4-CF_3$ | 1 | 171-173 | b |
| 26 | $2-Cl, 5-CF_3$ | 2 | 116-118 | g |
| 27 | $3-CF_3, 4-Cl$ | 2 | 204-206 | b |
| 28 | $3,5-(CF_3)_2$ | 2 | 174-176 | c |
| 29 | $-CH=CH-CH=CH-$ (2,3-Stellg.) | 2 | 146 | a |
| 30 | $4-OC_2H_5$ | 1 | 122 | a |
| 31 | $4-OC_3H_7-n$ | 1 | 114-116 | a |
| 32 | $3,5-(CH_3)_2$ | 2 | 150-152 | a |
| 33 | $4-NH-COCH_3$ | 1 | 203-205 | f |

Tabelle 2

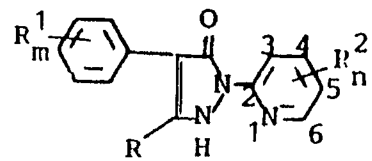

| Beisp.-Nr. | $R^1$ | m | R | $R^2$ | n | Schmelzpkt. (°C) | Umkristallisieren aus |
|---|---|---|---|---|---|---|---|
| 34 | - | 0 | $CH_3$ | - | 0 | 114-116 | a |
| 35 | - | 0 | $C_2H_5$ | - | 0 | 72- 73 | g |
| 36 | - | 0 | H | 6-Cl | 1 | 155-157 | b |
| 37 | 2-F, 6-Cl | 2 | H | 6-Cl | 1 | 220-221 | f |
| 38 | 3,4-$Cl_2$ | 2 | H | 6-Cl | 1 | 207 | f |
| 39 | 4-$CF_3$ | 1 | H | 6-Cl | 1 | 160-161 | b |
| 40 | - | 0 | H | 5-Cl | 1 | 209-211 | f |
| 41 | 2-Cl | 1 | H | 5-Cl | 1 | 195-197 | b |
| 42 | 3,4-$Cl_2$ | 2 | H | 5-Cl | 1 | 210-211 | b |
| 43 | 2-$OCH_3$ | 1 | H | 5-Cl | 1 | 176-177 | b |
| 44 | 2-$CH_3$ | 1 | H | 5-Cl | 1 | 188-190 | b |
| 45 | 3-$CH_3$ | 1 | H | 5-Cl | 1 | 156-158 | b |
| 46 | 4-$CH_3$ | 1 | H | 5-Cl | 1 | 207 | b |
| 47 | 4-$CF_3$ | 1 | H | 5-Cl | 1 | 218-220 | f |

<u>Tabelle 2</u> (Fortsetzung)

| Beisp.-Nr. | $R^1$ | m | R | $R^2$ | n | Schmelzpkt. (°C) | Umkristall. aus |
|---|---|---|---|---|---|---|---|
| 48 | - | 0 | $CH_3$ | 5-Cl | 1 | 156-157 | g |
| 49 | - | 0 | H | 5-Cl | 1 | 164-166 | a |
| 50 | 2-Cl | 1 | H | 3,5-$Cl_2$ | 2 | 198-199 | a |
| 51 | 3,4-$Cl_2$ | 2 | H | 3,5-$Cl_2$ | 2 | 112-114 | f |
| 52 | 2-$CH_3$ | 1 | H | 3,5-$Cl_2$ | 2 | 164-166 | c |
| 53 | 2-$OCH_3$ | 1 | H | 5-Br | 1 | 184-186 | b |
| 54 | - | 0 | H | 5-$NO_2$ | 1 | 264 (Zers.) | f |
| 55 | 4-$CH_3$ | 1 | H | 5-$NO_2$ | 1 | 267-268 | h |
| 56 | 4-$CF_3$ | 1 | H | 5-$NO_2$ | 1 | 196-198 | i und d |
| 57 | - | 0 | H | 6-$CH_3$ | 1 | 110-112 | g |
| 58 | - | 0 | H | 4-$CH_3$ | 1 | 171-173 | b |
| 59 | - | 0 | H | 4,6-$(CH_3)_2$ | 2 | 147-149 | g |
| 60 | - | 0 | H | 4-Cl,6-$CH_3$ | 2 | 214-216 | a |
| 61 | 4-$CF_3$ | 1 | H | 4-Cl,6-$CH_3$ | 2 | 208-210 | a |

<u>Tabelle 2</u> (Fortsetzung)

| Beisp.-Nr. | $R^1$ | m | R | $R^2$ | n | Schmelzpkt. (°C) | Umkristall. aus |
|---|---|---|---|---|---|---|---|
| 62 | - | 0 | H | 5-$CF_3$ | 1 | 172-174 | a |
| 63 | - | 0 | H | 5-CN | 1 | 243-245 | b |
| 64 | - | 0 | H | 3-CN,6-$CH_3$ | 2 | 300 | a |
| 65 | 2-Cl | 1 | H | 5-CO-$NH_2$ | 1 | 270 | h |
| 66 | - | 0 | H | -CH=CH-CH=CH-; 4-$CH_3$ (5,6-Stellg.) | 3 | 180-182 | b |
| 67 | 3,4-$Cl_2$ | 2 | H | -CH=CH-CH=CH-; 4-$CH_3$ (5,6-Stellg.) | 3 | 228-230 | f |
| 68 | 4-$CH_3$ | 1 | H | -CH=CH-CH=CH-; 4-$CH_3$ (5,6-Stellg.) | 3 | 196 | b |
| 69 | 4-$CF_3$ | 1 | H | -CH=CH-CH=CH-; 4-$CH_3$ (5,6-Stellg.) | 3 | 252-254 | b |
| 70 | 4-$OCH_3$ | 1 | -$CH_3$ | - | 0 | 126-128 | g |
| 71 | 2-F | 1 | -$CH_3$ | - | 0 | 98-100 | g |
| 72 | 4-$OCH_3$ | 1 | -$CH_3$ | 5-Cl | 1 | 180-181 | c |
| 73 | 4-$CH_3$ | 1 | -$CH_3$ | - | 0 | 114-115 | g |
| 74 | 3,4-$(Cl)_2$ | 2 | -$CH_3$ | - | 0 | 128-130 | g |
| 75 | 4-$OCH_3$ | 1 | H | 5-Cl | 1 | 167-169 | c |
| 76 | — | 0 | -$CH_3$ | 4-$CH_3$ | 1 | 144-145 | g |
| 77 | 4-$OCH_3$ | 1 | H | 4-$CH_3$ | 1 | 146-148 | g |

Tabelle 3

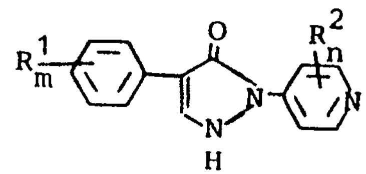

| Beisp.-Nr. | $R^1$ | m | $R^2$ | n | Schmelzpunkt (°C) | Umkristall. aus |
|---|---|---|---|---|---|---|
| 78 | - | 0 | - | 0 | 242-246 | i |
| 79 | - | 0 | 2,6-$(CH_3)_2$ | 2 | 254 | i |
| 80 | 4-$CF_3$ | 1 | 2,6-$(CH_3)_2$ | 2 | 260-264 | b |

Tabelle 4

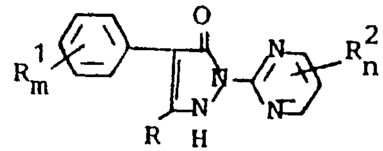

| Beisp.-Nr. | $R^1$ | m | R | $R^2$ | n | Schmelzpunkt (°C) | Umkristall. aus |
|---|---|---|---|---|---|---|---|
| 81 | - | 0 | H | - | 0 | 158-160 | a |
| 82 | 2-F | 1 | H | - | 0 | 168-170 | a |
| 83 | 4-F | 1 | H | - | 0 | 210-212 | a |
| 84 | 2-Cl | 1 | H | - | 0 | 140-150 | a |
| 85 | 4-Cl | 1 | H | - | 0 | 190-191 | d |
| 86 | 2-Br | 1 | H | - | 0 | 137-138 | a |
| 87 | 2-F, 6-Cl | 2 | H | - | 0 | 178 | b |
| 88 | 2,4-$Cl_2$ | 2 | H | - | 0 | 243-244 | b |
| 89 | 3,4-$Cl_2$ | 2 | H | - | 0 | 246-247 | f |
| 90 | 2-$OCH_3$ | 1 | H | - | 0 | 130 | a |
| 91 | 4-$OCH_3$ | 1 | H | - | 0 | 166-168 | c |
| 92 | 3-$OCF_3$ | 1 | H | - | 0 | 162-164 | c |

Tabelle 4 (Fortsetzung)

| Beisp.-Nr. | $R^1$ | m | R | $R^2$ | n | Schmelzpunkt (°C) | Umkristall. aus |
|---|---|---|---|---|---|---|---|
| 93 | 4-$OCF_3$ | 1 | H | - | 0 | 170-172 | a |
| 94 | 4-$SCF_3$ | 1 | H | - | 0 | 202-204 | b |
| 95 | -O-$CF_2$-O- (3,4-Stellg.) | 2 | H | - | 0 | 230-231 | f |
| 96 | 2-$CH_3$ | 1 | H | - | 0 | 133-134 | b |
| 97 | 3-$CH_3$ | 1 | H | - | 0 | 156-158 | a |
| 98 | 4-$CH_3$ | 1 | H | - | 0 | 168-170 | a |
| 99 | 3-$CF_3$ | 1 | H | - | 0 | 180-181 | b |
| 100 | 4-$CF_3$ | 1 | H | - | 0 | 226 | b |
| 101 | 2-$CF_3$, 4-Cl | 2 | H | - | 0 | 150-152 | a |
| 102 | 2-Cl; 5-$CF_3$ | 2 | H | - | 0 | 182-184 | c |
| 103 | 3-$CF_3$; 4-Cl | 2 | H | - | 0 | 188-189 | a |
| 104 | 3,5-$(CF_3)_2$ | 2 | H | - | 0 | 188-189 | a |
| 105 | -CH=CH-CH=CH- (2,3-Stellg.) | 2 | H | - | 0 | 146-148 | b |

<u>Tabelle 4</u> (Fortsetzung)

| Beisp.-Nr. | $R^1$ | m | R | $R^2$ | n | Schmelzpunkt (°C) | Umkristall. aus |
|---|---|---|---|---|---|---|---|
| 106 | - | 0 | $CH_3$ | - | 0 | 202-204 | b |
| 107 | - | 0 | H | 5-F | 1 | 245-246 | f |
| 108 | - | 0 | H | 5-Cl | 1 | 267-268 | i |
| 109 | 2-Cl | 1 | H | 5-Cl | 1 | 216-218 | b |
| 110 | 3,4-$Cl_2$ | 2 | H | 5-Cl | 1 | 242-244 | f |
| 111 | 2-$CH_3$ | 1 | H | 5-Cl | 1 | 204 | b |
| 112 | 4-$CH_3$ | 1 | H | 5-Cl | 1 | 264 | b |
| 113 | 4-$CF_3$ | 1 | H | 5-Cl | 1 | 268-270 | f |
| 114 | - | 0 | H | 6-$CH_3$ | 1 | 204-206 | a |
| 115 | - | 0 | H | 4,6-$(CH_3)_2$ | 2 | 256 | c |
| 116 | 4-F | 1 | H | 4,6-$(CH_3)_2$ | 2 | 275 | b |
| 117 | 4-Cl | 1 | H | 4,6-$(CH_3)_2$ | 2 | 270 | h |
| 118 | - | 0 | $CH_3$ | 4,6-$(CH_3)_2$ | 2 | 181-182 | c |
| 119 | 4-$OC_2H_5$ | 1 | H | - | 0 | 191-193 | b |
| 120 | 3,5-$(CH_3)_2$ | 2 | H | - | 0 | 110-112 | e |

Tabelle 5

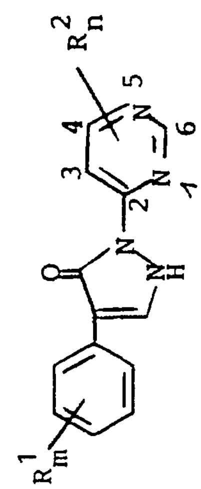

| Beisp.-Nr. | $R^1$ | m | $R^2$ | n | Schmelzpunkt (°C) | Umkristall. aus |
|---|---|---|---|---|---|---|
| 121 | - | 0 | 4,6-$(CH_3)_2$ | 2 | 192 | b |
| 122 | 4-$CF_3$ | 1 | 4,6-$(CH_3)_2$ | 2 | 196-198 | i |
| 123 | - | 0 | 4-Cl, 6-$CH_3$ | 2 | 162-164 | g |
| 124 | - | 0 | 3-$OCH_3$,6-t-$C_4H_9$ | 2 | 175 | b |
| 125 | 3,4-$Cl_2$ | 2 | 3-$OCH_3$,6-t-$C_4H_9$ | 2 | 196-197 | b |
| 126 | 4-$CF_3$ | 1 | 3-$OCH_3$,6-t-$C_4H_9$ | 2 | 200 | b |
| 127 | - | 0 | 4-$CH_3$,6-$SCH_3$ | 2 | 180-182 | c |
| 128 | 4-$CF_3$ | 1 | 4-$CH_3$,6-$SCH_3$ | 2 | 174-176 | g |

Anwendungsbeispiele

In den folgenden Anwendungsbeispielen werden die nachfolgend aufgeführten Verbindungen als Vergleichssubstanzen eingesetzt:

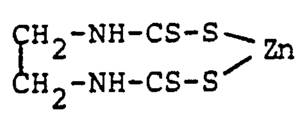

(A)

Zinkethylen-1,2-bis-(dithiocarbamat)

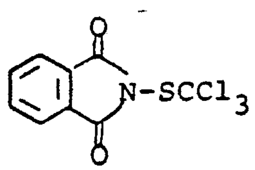

(B)

N-Trichlormethylthio-phthalimid

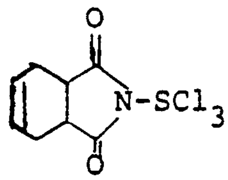

(C)

N-Trichlormethylthio-tetrahydrophthalimid

$$(CH_3)_2N-SO_2-N-C_6H_5$$
$$SCCl_2F$$

(D)

N,N-Dimethyl-N'-phenyl-N'-dichlorfluormethylthio-sulfamid

## Beispiel A

Phytophthora-Test (Tomate) /protektiv

Lösungsmittel: 4,7 Gewichtsteile ACETON
Emulgator: 0,3 Gewichtsteile ALKYL-ARYL-POLYGLYKOLETHER

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20° C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 100, 13, 105, 102, 93 und 92.

## Beispiel B

Botrytis-Test (BOHNE) / protektiv

Lösungsmittel: 4,7 Gewichtsteile ACETON
Emulgator: 0,3 Gewichtsteile ALKYL-ARYL-POLYGLYKOLETHER

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20° C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß den Herstellungsbeispielen: 84, 89, 11, 87, 12, 88, 96, 111, 21, 94 und 102.

Beispiel C

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach den Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25° C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 80, 122, 25, 68, 84, 105, 28, 11, 7, 102 und 104.

Beispiel D

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25° C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 105, 97, 18, 21, 102 und 94.

Beispiel E

Leptosphaeria nodorum-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20° C und 100% relativer Luftfeuchtigkeit in einer Inkubationskabine.

Die Pflanzen werden in einem Gewächshaus bei einer Temperatur von ca. 15° C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 19, 24, 5, 9, 26, 28, 15, 18, 81, 106 und 100.

Beispiel F

Cochliobolus sativus-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 12, 21, 24, 5, 9, 26, 18, 28, 100, 92, 85.

Beispiel G

Drechslera graminea-Test (Gerste) / Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4 °C aus. Dabei wird die Keimung der Gerste und gegebenenfalls auch der Pilzsporen eingeleitet. Anschließend sät man die vorgekeimte Gerste mit 2 x 50 Korn 3 cm tief in eine Standarderde und kultiviert sie im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigt bei diesem Test z.B. die Verbindung gemäß dem Herstellungsbeispiel: 21.

Beispiel H

Zum Nachweis der Wirksamkeit gegen Pilze werden die minimalen Hemm-Konzentrationen (MHK) von erfindungsgemäßen Wirkstoffen bestimmt:

Ein Agar, der aus Malzextrakt und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach zweiwöchiger Lagerung bei 28 °C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt, sie ist in der nachstehenden Tabelle angegeben.

Tabelle 1

MHK's in mg/l bei der Einwirkung erfindungsgemäßer Substanzen auf Pilze

| Testorganismen | gem.Bsp. 4 | 5 | 15 | 21 | 23 | 81 | 83 | 84 | 85 | 86 | 90 | 91 | 97 | 100 |
|---|---|---|---|---|---|---|---|---|---|---|---|---|---|---|
| Alternaria tenuis | 50 | 20 | 50 | 10 | 75 | 75 | 50 | 50 | 50 | 20 | 75 | 100 | 50 | 50 |
| Aspergillus niger | 7,5 | 3,5 | 20 | 7,5 | 7,5 | 10 | 15 | 10 | 5 | 10 | 35 | 50 | 10 | 500 |
| Aureobasidium pullulans | 100 | 20 | 50 | 35 | 50 | 50 | 50 | 20 | 50 | 20 | 75 | 150 | 50 | 50 |
| Chaetomium globosum | 1,5 | 0,5 | 0,5 | 0,75 | 0,5 | 3,5 | 3,5 | 1,5 | 1 | 1 | 15 | 20 | 1 | 1 |
| Coniophora puteana | 5 | 2 | 5 | 5 | 35 | 0,5 | 5 | 2 | 3,5 | 5 | 5 | 5 | 2 | 5 |
| Lentinus tigrinus | 75 | 20 | 50 | 50 | 75 | 5 | 50 | 50 | 7,5 | 2 | 100 | 100 | 10 | 15 |
| Penicillium glaucum | 10 | 5 | 75 | 7,5 | 5 | 50 | 20 | 20 | 10 | 50 | 35 | 10 | 10 | 10 |
| Polyporus versicolor | 35 | 7,5 | 20 | 7,5 | 3,5 | 2 | 35 | 35 | 5 | 2 | 10 | 20 | 5 | 5 |
| Sclerophoma pityophila | 10 | 15 | 50 | 35 | 20 | 7,5 | 5 | 10 | 20 | 10 | 15 | 15 | 20 | 20 |
| Trichoderma viride | 200 | 35 | 1000 | 50 | 1000 | 75 | 75 | 35 | 100 | 35 | 150 | 200 | 75 | 500 |

Beispiel J (Wirkung gegen Schleimorganismen)

Erfindungsgemäße Substanzen werden in Konzentrationen von jeweils 0,1 bis 100 mg/l in Allens Nährlösung (Arch. Mikrobiol. 17, 34 bis 53 (1952)), die in 4 l sterilem Wasser, 0,2 g Ammoniumchlorid, 4,0

g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach dreiwöchiger Kultur bei Raumtemperatur noch völlig klar, d.h. die in wirkstofffreien Nährlösungen nach 3 bis 4 Tagen bemerkbare starke Vermehrung der Mikroben und Schleimbildung unterbleibt.

Tabelle

MHK-Werte in mg/l bei der Einwirkung der unten angegebenen Substanzen auf Schleimorganismen

| Wirkstoff gemäß Beispiel | MHK in mg/l |
|---|---|
| 4 | 15 |
| 23 | 7,5 |
| 83 | 35 |

## Patentansprüche

1. 1-Heteroaryl-4-aryl-pyrazolin-5-one der Formel (I)

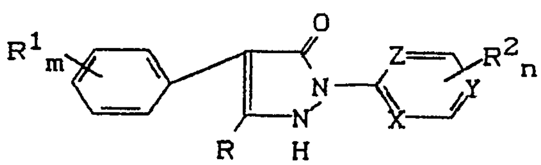

(I)

in welcher

| | |
|---|---|
| R | für Wasserstoff, Methyl oder Ethyl steht, |
| $R^1$ | für Fluor, Chlor, Brom, Methyl, Ethyl, n-Propyl, iso-Propyl, Trifluormethyl, Dichlorfluormethyl, Methoxy, Ethoxy, n-Propoxy, iso-Propoxy, Trifluormethoxy, Dichlorfluormethoxy, Methylthiomethoxy, Ethylthiomethoxy, 1-Methylthio-ethoxy, 2-Ethylthio-ethoxy, Phenoxymethoxy, Phenoxy, Methylthio, Ethylthio, Trifluormethylthio, Dichlorfluormethylthio, Methoxymethylthio, Ethoxymethylthio, Methylthiomethylthio, Methylsulfinyl, Ethylsulfinyl, Methylsulfonyl, Nitro, Cyano, Amino, Methylamino, Ethylamino, Dimethylamino, Diethylamino, Acetylamino, für einen ankondensierten Benzolring oder einen gegebenenfalls durch Fluor einfach oder mehrfach substituierten ankondensierten 5-oder 6-gliedrigen Heterocyclus mit 1 oder 2 Sauerstoffatomen steht, |
| $R^2$ | für Fluor, Chlor, Brom, Methyl, Ethyl, n-Propyl, iso-Propyl, tert.-Butyl, Trifluormethyl, Dichlorfluormethyl, Methoxymethyl, Ethoxymethyl, Methylthiomethyl, Methoxy, Ethoxy, n-Propoxy, iso-Propoxy, Methylthio, Ethylthio, Nitro, Cyano, Carbonsäureamid oder einen ankondensierten Benzolring steht, |
| m | für eine ganze Zahl 0 bis 3 steht, |
| n | für eine ganze Zahl von 0 bis 3 steht, wobei die Substituenten gleich oder verschieden sein können, wenn m und/oder n für eine Zahl größer 1 stehen und |
| X, Y und Z | gleich oder verschieden sind und für ein Stickstoffatom, den Rest =CH- oder |

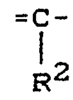

stehen, wobei $R^2$ die oben angegebene Bedeutung hat, mit der Maßgabe, daß wenigstens einer der Reste X, Y, Z ein Stickstoffatom ist.

**2.** Verfahren zur Herstellung von 1-Heteroaryl-4-aryl-pyrazolin-5-one der Formel I

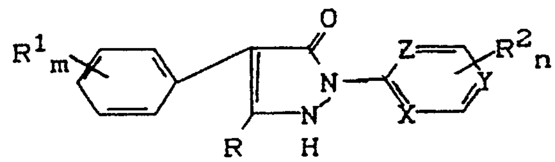 (I)

in welcher

| | |
|---|---|
| R | für Wasserstoff, Methyl oder Ethyl steht, |
| $R^1$ | für Fluor, Chlor, Brom, Methyl, Ethyl, n-Propyl, iso-Propyl, Trifluormethyl, Dichlorfluormethyl, Methoxy, Ethoxy, n-Propoxy, iso-Propoxy, Trifluormethoxy, Dichlorfluormethoxy, Methylthiomethoxy, Ethylthiomethoxy, 1-Methylthio-ethoxy, 2-Ethylthio-ethoxy, Phenoxymethoxy, Phenoxy, Methylthio, Ethylthio, Trifluormethylthio, Dichlorfluormethylthio, Methoxymethylthio, Ethoxymethylthio, Methylthiomethylthio, Methylsulfinyl, Ethylsulfinyl, Methylsulfonyl, Nitro, Cyano, Amino, Methylamino, Ethylamino, Dimethylamino, Diethylamino, Acetylamino, für einen ankondensierten Benzolring oder einen gegebenenfalls durch Fluor einfach oder mehrfach substituierten ankondensierten 5-oder 6-gliedrigen Heterocyclus mit 1 oder 2 Sauerstoffatomen steht, |
| $R^2$ | für Fluor, Chlor, Brom, Methyl, Ethyl, n-Propyl, iso-Propyl, tert.-Butyl, Trifluormethyl, Dichlorfluormethyl, Methoxymethyl, Ethoxymethyl, Methylthiomethyl, Methoxy, Ethoxy, n-Propoxy, iso-Propoxy, Methylthio, Ethylthio, Nitro, Cyano, Carbonsäureamid oder einen ankondensierten Benzolring steht, |
| m | für eine ganze Zahl 0 bis 3 steht, |
| n | für eine ganze Zahl von 0 bis 3 steht, wobei die Substituenten gleich oder verschieden sein können, wenn m und/oder n für eine Zahl größer 1 stehen und |
| X, Y und Z | gleich oder verschieden sind und für ein Stickstoffatom, den Rest =CH- oder |

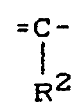

stehen, wobei $R^2$ die oben angegebene Bedeutung hat, mit der Maßgabe, daß wenigstens einer der Reste X, Y, Z ein Stickstoffatom bedeutet,

dadurch gekennzeichnet, daß man α-Acylphenylessigsäureester oder deren Derivate der Formel (II)

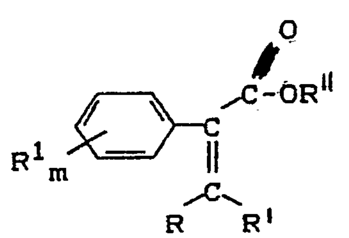 (II)

in welcher

| | |
|---|---|
| R, $R^1$ und m | die unter Formel (I) angegebenen Bedeutungen haben, |
| R' | für Hydroxy, Alkoxy, Halogen, Dialkylamino oder für die $-O-SO_2R'''$ Gruppe steht, |
| R" | für Alkyl oder gegebenenfalls substituiertes Aryl stehen und |
| R''' | für gegebenenfalls substituiertes Alkyl, gegebenenfalls substituiertes Aryl oder Dialkylamino steht, |

mit Hydrazinoheterocyclen der Formel (III)

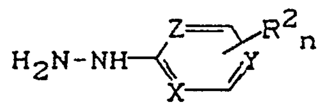

(III)

in welcher

| | |
|---|---|
| X, Y, Z, $R^2$ und n | die unter Formel (I) angegebenen Bedeutungen haben, gegebenenfalls in Gegenwart eines Lösungsmittels und gegebenenfalls in Gegenwart einer Base oder Säure umsetzt. |

3. Mikrobizide Mittel, gekennzeichnet durch einen Gehalt an mindestens einem 1-Heteroaryl-4-aryl-pyrazolin-5-on der Formel (I) gemäß Anspruch 1.

4. Verfahren zur Bekämpfung von Mikroben, dadurch gekennzeichnet, daß man 1-Heteroaryl-4-aryl-pyrazolin-5-one der Formel (I) gemäß Anspruch 1 auf Mikroben und/oder ihren Lebensraum einwirken läßt.

5. Verwendung von 1-Heteroaryl-4-aryl-pyrazolin-5-onen der Formel (I) gemäß den Anspruchen 1 zur Bekämpfung von Mikroben.

6. Verfahren zur Herstellung von mikrobiziden Mitteln, dadurch gekennzeichnet, daß man 1-Heteroaryl-4-aryl-pyrazolin-5-one der Formel (I) gemäß Anspruch 1 mit Streckmitteln und/oder oberflächenaktiven Mittel vermischt.

7. Mikrobizide Mittel gemäß Anspruch 3 zum Schutz technischer Materialien und zum Einsatz im Pflanzenschutz.

8. Verwendung von 1-Heteroaryl-4-aryl-pyrazolin-5-onen der Formel (I) gemäß Anspruch 5 zur Bekämpfung von Miokroben im Pflanzenschutz und in technischen Materialien.

## Claims

1. 1-Heteroaryl-4-aryl-pyrazolin-5-ones of the formula (I)

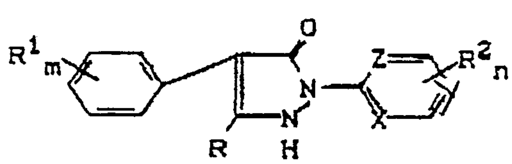

(I)

in which

| | |
|---|---|
| R | represents hydrogen, methyl or ethyl, |
| $R^1$ | represents fluorine, chlorine, bromine, methyl, ethyl, n-propyl, isopropyl, trifluoromethyl, dichlorofluoromethyl, methoxy, ethoxy, n-propoxy, isopropoxy, trifluoromethoxy, dichlorofluoromethoxy, methylthiomethoxy, ethylthiomethoxy, 1-methylthio-ethoxy, 2-ethylthio-ethoxy, phenoxy-methoxy, phenoxy, methylthio, ethylthio, trifluoromethylthio, dichlorofluoromethylthio, methoxymethylthio, ethoxymethylthio, methylthiomethylthio, methylsulphinyl, ethylsulphinyl, methylsulphonyl, nitro, |

cyano, amino, methylamino, ethylamino, dimethylamino, diethylamino, a fused-on benzene ring or a fused-on 5- or 6-membered heterocyclic structure which has 1 or 2 oxygen atoms and is optionally monosubstituted or polysubstituted by fluorine,

$R^2$ represents fluorine, chlorine, bromine, methyl, ethyl, n-propyl, isopropyl, tert.-butyl, trifluoromethyl, dichlorofluoromethyl, methoxymethyl, ethoxymethyl, methylthiomethyl, methoxy, ethoxy, n-propoxy, isopropoxy, methylthio, ethylthio, nitro, cyano, carboxamide or a fused-on benzene ring,

m represents an integer from 0 to 3,

n represents an integer from 0 to 3, it being possible for the substituents to be identical or different when m and/or n represent a number greater than 1, and

X, Y and Z are identical or different and represent a nitrogen atom or the radical =CH- or

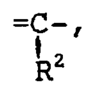

wherein $R^2$ has the meaning given above, with the proviso that at least one of the radicals X, Y and Z is a nitrogen atom.

2. Process for the preparation of 1-heteroaryl-4-aryl-pyrazolin-5-ones of the formula I

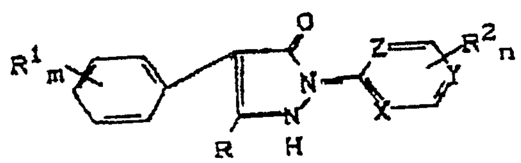

(I)

in which

R represents hydrogen, methyl or ethyl,

$R^1$ represents fluorine, chlorine, bromine, methyl, ethyl, n-propyl, isopropyl, trifluoromethyl, dichlorofluoromethyl, methoxy, ethoxy, n-propoxy, isopropoxy, trifluoromethoxy, dichlorofluoromethoxy, methylthiomethoxy, ethylthiomethoxy, 1-methylthio-ethoxy, 2-ethylthio-ethoxy, phenoxy-methoxy, phenoxy, methylthio, ethylthio, trifluoromethylthio, dichlorofluoromethylthio, methoxymethylthio, ethoxymethylthio, methylthiomethylthio, methylsulphinyl, ethylsulphinyl, methylsulphonyl, nitro, cyano, amino, methylamino, ethylamino, dimethylamino, diethylamino, a fused-on benzene ring or a fused-on 5- or 6-membered heterocyclic structure which has 1 or 2 oxygen atoms and is optionally monosubstituted or polysubstituted by fluorine,

$R^2$ represents fluorine, chlorine, bromine, methyl, ethyl, n-propyl, isopropyl, tert.-butyl, trifluoromethyl, dichlorofluoromethyl, methoxymethyl, ethoxymethyl, methylthiomethyl, methoxy, ethoxy, n-propoxy, isopropoxy, methylthio, ethylthio, nitro, cyano, carboxamide or a fused-on benzene ring,

m represents an integer from 0 to 3,

n represents an integer from 0 to 3, it being possible for the substituents to be identical or different when m and/or n represent a number greater than 1, and

X, Y and Z are identical or different and represent a nitrogen atom or the radical =CH- or

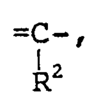

wherein

$R^2$ has the meaning given above, with the proviso that at least one of the radicals X, Y and Z denotes a nitrogen atom,

characterised in that α-acylphenylacetates or their derivatives of the formula (II)

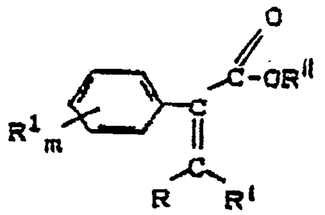

(II)

in which

R, $R^1$ and m have the meanings given under formula (I),

R' represents hydroxyl, alkoxy, halogen, dialkylamino or the $-O-SO_2R'''$ group,

R'' represents alkyl or optionally substituted aryl and

R''' represents optionally substituted alkyl, optionally substituted aryl or dialkylamino, are reacted with hydrazinoheterocycles of the formula (III)

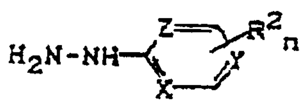

(III)

in which

x, Y, Z, $R^2$ and n have the meanings given under formula (I), if appropriate in the presence of a solvent and, if appropriate, in the presence of a base or an acid.

3. Microbicidal agents, characterised in that they contain at least one 1-heteroaryl-4-aryl-pyrazolin-5-one of the formula (I) according to Claim 1.

4. Method of combating microbes, characterised in that 1-heteroaryl-4-aryl-pyrazolin-5-ones of the formula (I) according to Claim 1 are allowed to act on microbes and/or their habitat.

5. Use of 1-heteroaryl-4-aryl-pyrazolin-5-ones of the formula (I) according to Claim 1 for combating microbes.

6. Process for the preparation of microbicidal agents, characterised in that 1-heteroaryl-4-aryl-pyrazolin-5-ones of the formula (I) according to Claim 1 are mixed with extenders and/or surface-active agents.

7. Microbicidal agents according to Claim 3 for the protection of industrial materials and for use in crop protection.

8. Use of 1-heteroaryl-4-aryl-pyrazolin-5-ones of the formula (I) according to Claim 5 for combating microbes in crop protection and in industrial materials.

## Revendications

1. 1-hétéroaryl-4-aryl-pyrazoline-5-ones de formule (I)

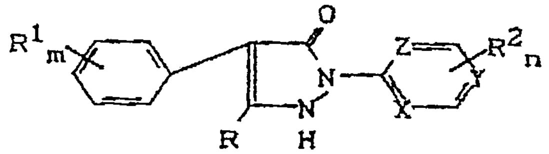

(I)

dans laquelle

R est l'hydrogène, un groupe méthyle ou éthyle,

R¹ est le fluor, le chlore, le brome, un groupe méthyle, éthyle, n-propyle, isopropyle, trifluorométhyle, dichlorofluorométhyle, méthoxy, éthoxy, n-propoxy, isopropoxy, trifluorométhoxy, dichlorofluorométhoxy, méthylthiométhoxy, éthylthiométhoxy, 1-méthylthio-éthoxy, 2-éthylthio-éthoxy, phénoxyméthoxy, phénoxy, méthylthio, éthylthio, trifluorométhylthio, dichlorofluorométhylthio, méthoxyméthylthio, éthoxyméthylthio, méthylthiométhylthio, méthylsulfinyle, éthylsulfinyle, méthylsulfonyle, nitro, cyano, amino, méthylamino, éthylamino, diméthylamino, diéthylamino, acétylamino, un noyau benzénique condensé ou un hétérocycle pentagonal ou hexagonal comportant 1 ou 2 atomes d'oxygène, éventuellement substitué une ou plusieurs fois par du fluor,

$R^2$ représente le fluor, le chlore, le brome, un groupe méthyle,, éthyle, n-propyle, isopropyle, tertio-butyle, trifluorométhyle, dichlorofluorométhyle, méthoxyméthyle, éthoxyméthyle, méthylthiométhyle, méthoxy, éthoxy, n-propoxy, isopropoxy, méthylthio, éthylthio, nitro, cyano, amide d'acide carboxylique ou un noyau benzénique condensé,

m est un nombre entier de 0 à 3,

n est un nombre entier de 0 à 3, les substituants pouvant être identiques ou différents lorsque m et/ou n représentent un nombre supérieur à 1 et

X, Y et Z sont identiques ou différents et représentent un atome d'azote, le reste =CH ou

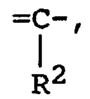

où $R^2$ a la définition indiquée ci-dessus, sous réserve qu'au moins l'un des restes X, Y, Z soit un atome d'azote.

**2.** Procédé de production de 1-hétéroaryl-4-aryl-pyrazoline-5-ones de formule I

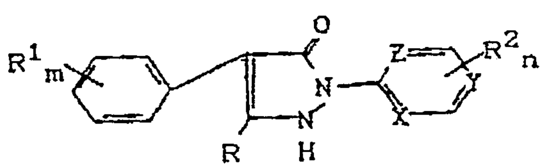

(I)

dans laquelle

R est l'hydrogène, un groupe méthyle ou éthyle,

R¹ est le fluor, le chlore, le brome, un groupe méthyle, éthyle, n-propyle, isopropyle, trifluorométhyle, dichlorofluorométhyle, méthoxy, éthoxy, n-propoxy, isopropoxy, trifluorométhoxy, dichlorofluorométhoxy, méthylthiométhoxy, éthylthiométhoxy, 1-méthylthio-éthoxy, 2-éthylthio-éthoxy, phénoxyméthoxy, phénoxy, méthylthio, éthylthio, trifluorométhylthio, dichlorofluorométhylthio, méthoxyméthylthio, éthoxyméthylthio, méthylthiométhylthio, méthylsulfinyle, éthylsulfinyle, méthylsulfonyle, nitro, cyano, amino, méthylamino, éthylamino, diméthylamino, diéthylamino, acétylamino, un noyau benzénique condensé ou un hétérocycle pentagonal ou hexagonal comportant 1 ou 2 atomes d'oxygène, éventuellement substitué une ou plusieurs fois par du fluor,

$R^2$ représente le fluor, le chlore, le brome, un groupe méthyle, éthyle, n-propyle, isopropyle, tertio-butyle, trifluorométhyle, dichlorofluorométhyle, méthoxyméthyle, éthoxyméthyle, méthylthiométhyle, méthoxy, éthoxy, n-propoxy, isopropoxy, méthylthio, éthylthio, nitro, cyano, amide d'acide carboxylique ou un noyau benzénique condensé,

m est un nombre entier de 0 à 3,

n est un nombre entier de 0 à 3, les substituants pouvant être identiques ou différents lorsque m et/ou n représentent un nombre supérieur à 1 et

X, Y et Z sont identiques ou différents et représentent un atome d'azote, le reste =CH- ou

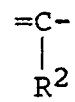

où $R^2$ a la définition indiquée ci-dessus, sous réserve qu'au moins l'un des restes X, Y, Z soit un atome d'azote,

caractérisé en ce qu'on fait réagir des esters d'acide α-acylphénylacétique ou leurs dérivés de formule (II)

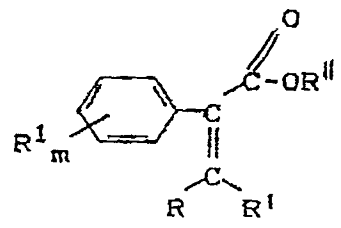 (II)

dans laquelle

- R, $R^1$ et m — ont les définitions indiquées pour la formule (I),
- R' — est un groupe hydroxy, alkoxy, un halogène, un groupe dialkylamino ou le groupe $-O-SO_2R'''$,
- R'' — est un groupe alkyle ou un groupe aryle éventuellement substitué et
- R''' — est un groupe alkyle éventuellement substitué, un groupe aryle éventuellement substitué ou un groupe dialkylamino,

avec des hydrazinohétérocycles de formule (III)

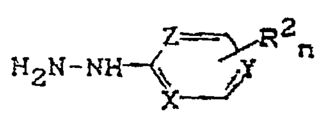 (III)

dans laquelle

- X, Y, Z, $R^2$ et n — ont les définitions indiquées pour la formule (I), le cas échéant en présence d'un solvant et en la présence éventuelle d'une base ou d'un acide.

3. Compositions microbicides, caractérisées par une teneur en au moins une 1-hétéroaryl-4-aryl-pyrazoline-5-one de formule (I) suivant la revendication 1.

4. Procédé pour combattre des microbes, caractérisé en ce qu'on fait agir des 1-hétéroaryl-4-aryl-pyrazoline-5-ones de formule (I) suivant la revendication 1 sur des microbes et/ou sur leur milieu.

5. Utilisation de 1-hétéroaryl-4-aryl-pyrazoline-5-ones de formule (I) suivant la revendication 1, pour combattre des microbes.

6. Procédé de préparation de compositions microbicides, caractérisé en ce qu'on mélange des 1-hétéroaryl-4-aryl-pyrazoline-5-ones de formule (I) suivant la revendication 1 avec des diluants et/ou des agents tensio-actifs.

7. Compositions microbicides suivant la revendication 3, destinées à la protection de matériaux industriels et à être utilisées dans la protection de plantes.

8. Utilisation de 1-hétéroaryl-4-aryl-pyrazoline-5-ones de formule (I) suivant la revendication 5 pour combattre des microbes dans la protection des plantes et dans des matériaux industriels.